# EUROPÄISCHE PATENTSCHRIFT

(11) **EP 1 360 955 B1**
(45) Veröffentlichungstag und Bekanntmachung des Hinweises auf die Patenterteilung: **15.09.2010**
(21) Anmeldenummer: 03010016.8
(22) Anmeldetag: 02.05.2003
(51) Int. Cl.: A61K 8/81, A61K 8/89, A61Q 5/00, A61Q 15/00, A61Q 19/00

(54) **Kosmetische Zusammensetzung mit einem Silicon-Elastomer und einem verdickenden Polymerlatex**
Cosmetic composition with a silicone elastomer and a thickening polymer in the latex form
Composition cosmétique avec un élastomère silicone et un latex de polymère épaississant

(30) Priorität: 10.05.2002 DE 10220867
(43) Veröffentlichungstag der Anmeldung: 12.11.2003
(73) Patentinhaber: Henkel AG & Co. KGaA, 40589 Düsseldorf (DE)
(72) Erfinder: Augustin-Castro, Barbara, Dr., 6291 XL Vaals (NL); Waldmann-Laue, Marianne, Dr., 40789 Monheim (DE); Blumenkamp, Elke, 41751 Viersen (DE)

(56) Entgegenhaltungen:
- EP-A1- 1 097 703
- EP-A2- 0 874 017
- EP-A2- 1 325 729
- WO-A1-99/42521
- US-A- 5 470 551
- US-A- 6 099 829
- US-A1- 2001 041 768
- US-B1- 6 274 151
- US-B1- 6 338 841
- DATABASE CHEMICAL ABSTRACTS [Online] retrieved from STN Database accession no. 129: 8 373 XP002263112 & T. ITOU ET AL.: "Application of silicone elastomer to hair styling agents" FRAGRANCE JOURNAL, Bd. 26, Nr. 5, 1998, Seiten 17-22, jp
- ANON.: "Estee Lauder . Re-Nutriv ultimate lifting creme" COSMETIC RESEARCH, March 2002 (2002-03), page 80, FR

## Beschreibung

Moderne kosmetische Zusammensetzungen zur Reinigung oder Pflege der Haut und der Haare müssen vielfachen Anforderungen des Verbrauchers genügen.
Neben der Erfüllung ihrer eigentlichen Bestimmung - gute Reinigungskraft bei Shampoos, Dusch- und Schaumbädern, Gesichtsreinigern, Make-up-Entfernern usw., gute konditionierende Wirkung ohne belastende Build-up-Effekte bei Haarconditionern und -kuren, Feuchtigkeitsbindung, Stärkung der Barrierefunktion der Haut und Verbesserung des Erscheinungsbildes der Haut bei Hautcremes, -gelen, -lotionen, -masken usw. sowie gute desodorierende oder schweißhemmende Wirkung von Deodorantien und Antitranspirantien - werden weitere kosmetische Effekte erwartet. Bei einem Reinigungsmittel kann dies beispielsweise eine haut- oder haarkonditionierende, feuchtigkeitsspendende oder rückfettende Wirkung sein, bei einer Hautcreme oder einer ähnlichen Zusammensetzung kann das die Faltenbehandlung, Hautaufhellung oder -bräunung, das Kaschieren von Hautunreinheiten und -unebenheiten sein, und von einem Deo- oder Antitranspirant-Produkt wird erwartet, daß es im Kontakt mit der Kleidung keine oder nur geringe Rückstände hinterlässt.

Ein weiteres wichtiges Kriterium bei der Beurteilung einer kosmetischen Zusammensetzung durch den Anwender ist der sensorische, insbesondere der taktil-sensorische Eindruck, der bei der Applikation der Zusammensetzung auf die Haut oder die Haare hervorgerufen wird.
Als besonders angenehm wird von vielen Verbrauchern der sensorische Eindruck, den Silicon-Verbindungen, z. B. Siliconöle oder Silicon-Elastomere erzeugen, empfunden. Siliconöle werden aufgrund ihres Spreit- und Einzugsverhaltens als samtweich und nichtfettend empfunden. Sie hinterlassen die Haut ohne Fettglanz und wirken nicht comedogen.

Bei höheren Anteilen an Siliconölen in einer kosmetischen Zusammensetzung können allerdings Schwierigkeiten bei der Einstellung der für das jeweilige Anwendungsgebiet geeigneten Viskosität auftreten, häufig in Verbindung mit Problemen bei der Lager- und/oder Temperaturstabilität.

EP 1325729 A1 offenbart kosmetische Zusammensetzungen, die ein 2-Methyl-2[(1-oxo-2-propenyl)amino]-1-propansulfonsäure-Polymer und ein lineares, unvemetztes Silicon-Blockcopolymer enthalten.

EP 1097703 A1 offenbart kosmetische Zusammensetzungen, die eine Kombination aus einer wässrigen Dispersion von Silicon-Elastomeren und einem Zucker oder Zuckerderivat enthalten, die mit einem Konsistenzregler versetzt werden können. Als geeigneter Konsistenzregler ist der Rohstoff Sepigel 305 von Seppic offenbart, der einen inversen Latex aus einem Copolymer mit einem Monomer mit einer starken Säurefunktion (2-Acrylamido-2-Methylpropansulfonsäure) und dem neutralen Monomer Acrylamid enthält. US 2001/041768 A1 offenbart kosmetische Zusammensetzungen, die mindestens ein emulgierendes Silicon-Elastomer mit mindestens einer Oxyalkylengruppe enthalten. Weiterhin sind verdickende Homopolymere aus einem Monomer mit einer starken Säurefunktion offenbart, die nicht in Form eines inversen, auto-inversiblen Polymerlatex mit einer Ölphase, einer Wasserphase und einem Öl-in-Wasser-Emulgator vorliegen. Gemäß Cosmetic Research, März 2002, enthält das Produkt "Re-Nutriv Ultimate Lifting Creme" von Estee Lauder ein Silicon-Elastomer und einen polymeren Verdicker in Form eines inversen, auto-inversiblen Polymerlatex, der eine Ölphase, eine Wasserphase, einen Öl-in-Wasser-Emulgator und einen vernetzten Polyelektrolyten enthält, der ausgewählt ist aus einem Copolymer, das Natrium-2-acrylamido-2-methylpropansulfonat und Acrylamid enthält.

Eine Aufgabe der vorliegenden Erfindung war die Bereitstellung einer ästhetisch ansprechenden kosmetischen Zusammensetzung mit hervorragenden pflegenden und konditionierenden Eigenschaften. Eine weitere Aufgabe der vorliegenden Erfindung war die Bereitstellung einer sensorisch, insbesondere taktil-sensorisch, ansprechenden kosmetischen Zusammensetzung zur kosmetischen Behandlung der Haut, zur pflegenden, verformenden, färbenden oder bleichenden Behandlung der Haare und zur desodorierenden und/oder schweißhemmenden Behandlung der Haut, insbesondere der Achselhöhlen. Eine weitere Aufgabe der vorliegenden Erfindung war die Bereitstellung einer lager- und/oder temperaturstabilen kosmetischen Zusammensetzung.

Überraschenderweise wurde nun gefunden, daß mit Hilfe einer Kombination aus mindestens einem Silicon-Elastomer und einem verdickenden Polymer in Form eines inversen, auto-inversiblen Latex lager- und temperaturstabile kosmetische Zusammensetzung mit einem außergewöhnlich angenehmen sensorischen, insbesondere taktil-sensorischen, Eindruck hergestellt werden können.

Unter einem autoinversiblen Polymerlatex ist erfindungsgemäß ein Polymerlatex zu verstehen, der erhältlich ist durch Emulsionspolymerisation der Monomeren, die in einer Wasser-in-Öl-Emulsion gestartet wird, am Ende der Polymerisationsreaktion aber durch die entstandenen Polymere in eine Öl-in-Wasser-Emulsion invertiert.

Ein erster Gegenstand der vorliegenden Erfindung ist eine kosmetische Zusammensetzung, die
a) mindestens ein Silicon-Elastomer das erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist und
b) mindestens einen polymeren Verdicker in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten, wobei der Polyelektrolyt ausgewählt ist aus teilweise oder vollständig neutralisierten, vernetzten Copolymeren aus 2-Methyl-2[(1-oxo-2-propenyl)amino]-1-propansulfonsäure (AMPS) und Hydroxyethylacrylat, in einem geeigneten kosmetischen Träger enthält.

Im Sinne der vorliegenden Erfindung ist das Polyelektrolytmonomer 2-Methyl-2[(1-oxo-2-propenyl)amino]-1-propansulfonsäure teilweise oder vollständig neutralisiert als Natrium- , Kalium-, Ammonium-, Ethanolamin- oder Aminosäure-Salz.

Erfindungsgemäße verdickende Polymere sind teilweise oder vollständig neutralisierte, vernetzte Copolymere aus 2-Methyl-2[(1-oxo-2-propenyl)-amino]-1-propansulfonsäure (AMPS) und Hydroxyethylacrylat, in Form eines inversen, auto-inversiblen Latex kommerziell erhältlich, z. B. unter dem Handelsnamen Simulgel^{®}NS von der Firma Seppic.

Der vorgenannte, erfindungsgemäß verwendete inverse Polymerlatex enthält mindestens ein Öl, ausgewählt aus weißen Mineralölen, Squalan und hydriertem Polyisobuten, sowie mindestens einen Öl-in-Wasser-Emulgator, ausgewählt aus den Ethylenoxid-Addukten von Sorbitanoleat, Ricinusöl, das gewünschtenfalls gehärtet ist, Sorbitanlaurat und Laurylalkohol.

Die erfindungsgemäß verwendeten verdickenden Polymerlatices weisen einen Polymergehalt von 30 - 90 Gew.%, bevorzugt 50 - 80 Gew.% und besonders bevorzugt 60 - 75 Gew.%, jeweils bezogen auf den gesamten Latex, auf. Bezogen auf die gesamte erfindungsgemäße kosmetische Zusammensetzung, wird das verdickende Polymer in Mengen von 0,1 - 5 Gew.%, bevozugt 0,3 - 3 Gew.% und besonders bevorzugt 0,5 - 2,5 Gew.%, bezogen auf die gesamte erfindungsgemäße kosmetische Zusammensetzung, eingesetzt.

Die erfindungsgemäß geeigneten Silicon-Elastomere sind erhältlich durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist.

Das Organopolysiloxan mit mindestens 2 C₂ - C₁₀ - Alkenyl-Gruppen mit terminaler Doppelbindung im Molekül ist ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

Das vernetzende Organopolysiloxan mit mindestens zwei Silicon-gebundenen Wasserstoffatomen ist ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren.

Erfindungsgemäß können sowohl nicht-emulgierende als auch emulgierende Silicon-Elastomere sowie deren Mischungen eingesetzt werden.
Geeignete nicht-emulgierende Silicon-Elastomere sind im Handel erhältlich, beispielsweise von Dow Corning die Produkte DC 9040, DC 9041 oder DC 9509, von General Electric, z. B. das Handelsprodukt SFE 839, von Shin-Etsu die Handelsprodukte KSG-15, KSG-16 oder KSG-18 sowie von Grant Industries die Produkte aus der Gransil®-Serie, wie z. B. Gransil®RPS Gel, INCI-Bezeichnung Cyclopentasiloxane and Polysilicone-11 oder Gransil®GCM-4, INCI-Bezeichnung Cyclotetrasiloxane and Polysilicone-11.

Geeignete emulgierende Silicon-Elastomere enthalten als funktionelle Gruppen am Polysiloxan-Gerüst Polyoxyethylen- und/oder Polyoxypropylen-Gruppen. Diese Gruppen können endständig und/oder als Seitengruppen zur Polysiloxan-Kette angeordnet sein. Geeignete emulgierende Silicon-Elastomere sind im Handel erhältlich, beispielsweise von Shin-Etsu die Produkte KSG-21, KSG-31, KSG-31X, KSG-32 oder von Dow Corning das Handelsprodukt DC-9011.

Der Gehalt an nicht-emulgierendem und/oder emulgierendem Silicon-Elastomer beträgt in den erfindungsgemäßen Zusammensetzungen 0,1 - 40 Gew.%, bevorzugt 0,5 - 20 Gew.% und besonders bevorzugt 1 - 10 Gew.%, jeweils bezogen auf die gesamte kosmetische Zusammensetzung.

Ein weiterer Gegenstand der vorliegenden Erfindung ist die Verwendung einer kosmetischen Zusammensetzung gemäß einem der Ansprüche 1 - 7 zur nicht-therapeutischen, kosmetischen Behandlung und/oder Minimierung von Hautfalten und -fältchen.

Ein weiterer Gegenstand der vorliegenden Erfindung ist die Verwendung einer kosmetischen Zusammensetzung gemäß einem der Ansprüche 1 - 7 zur pflegenden, verformenden, färbenden oder bleichenden Behandlung der Haare.

Ein weiterer Gegenstand der vorliegenden Erfindung ist die Verwendung einer kosmetischen Zusammensetzung gemäß einem der Ansprüche 1 - 7 zur desodorierenden und/oder schweißhemmenden Behandlung der Haut, insbesondere der Achselhöhlen.

In einer weiteren bevorzugten Ausführungsform enthalten die erfindungsgemäßen kosmetischen Zusammensetzungen mindestens einen organischen oder mineralischen oder modifizierten mineralischen Lichtschutzfilter. Bei den Lichtschutzfiltern handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist erfindungsgemäß bevorzugt.

Die erfindungsgemäß verwendeten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und - carbonsäureamiden, Ketotricyclo(5.2.1.0)decan, Benzalmalonsäureestern sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert.-Butylphenyl)-3-(4'-methoxyphenyl)propan-1,3-dion (Parsol® 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone (Uvasorb® HEB) sowie beliebige Mischungen der genannten Komponenten.

Bevorzugte wasserlösliche UV-Filter sind 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol® 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

In den erfindungsgemäßen Zusammensetzungen sind die Lichtschutzfilter in Mengen von 0,1 - 30 Gew.-%, bevorzugt 1 - 20 Gew.-% und besonders bevorzugt 2 - 15 Gew.-%, jeweils bezogen auf die gesamte Zusammensetzung, enthalten.

In einer bevorzugten Ausführungsform der vorliegenden Erfindung können die kosmetischen Zusammensetzungen weiterhin mindestens ein Proteinhydrolysat oder dessen Derivat enthalten. Erfindungsgemäß können sowohl pflanzliche als auch tierische Proteinhydrolysate eingesetzt werden. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin® (Diamalt), Gluadin® (Cognis), Lexein® (Inolex) und Crotein® (Croda).
Wenngleich der Einsatz der zusätzlichen Proteinhydrolysate als solche bevorzugt ist, können an ihrer Stelle gegebenenfalls auch anderweitig erhaltene Aminosäuregemische oder einzelne Aminosäuren wie beispielsweise Arginin, Lysin, Histidin oder Pyroglutaminsäure eingesetzt werden. Ebenfalls möglich ist der Einsatz von Derivaten der Proteinhydrolysate, z. B. in Form ihrer Fettsäure-Kondensationsprodukte. Entsprechende Handelsprodukte sind z. B. Lamepon® (Cognis), Gluadin® (Cognis), Lexein® (Inolex), Crolastin® oder Crotein® (Croda).
In einer bevorzugten Ausführungsform der vorliegenden Erfindung können die kosmetischen Zusammensetzungen weiterhin mindestens ein Mono-, Oligo- oder Polysaccharid oder deren Derivate enthalten. Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose sowie die Desoxyzucker Fucose und Rhamnose. Bevorzugt sind Glucose, Fructose, Galactose, Arabinose und Fucose; Glucose ist besonders bevorzugt.
Erfindungsgemäß geeignete Oligosaccharide sind aus zwei bis zehn Monosaccharideinheiten zusammengesetzt, z. B. Saccharose, Lactose oder Trehalose. Ein besonders bevorzugtes Oligosaccharid ist Saccharose. Ebenfalls besonders bevorzugt ist die Verwendung von Honig, der überwiegend Glucose und Saccharose enthält.
Erfindungsgemäß geeignete Polysaccharide sind aus mehr als zehn Monosaccharideinheiten zusammengesetzt. Bevorzugte Polysaccharide sind die aus α-D-Glucose-Einheiten aufgebauten Stärken sowie Stärkeabbauprodukte wie Amylose, Amylopektin und Dextrine. Erfindungsgemäß besonders vorteilhaft sind chemisch und/oder thermisch modifizierte Stärken, z. B. Hydroxypropylstärkephosphat, Dihydroxypropyldistärkephosphat oder die Handelsprodukte Dry Flo®. Weiterhin bevorzugt sind Dextrane sowie ihre Derivate, z. B. Dextransulfat. Ebenfalls bevorzugt sind nichtionische Cellulose-Derivate, wie Methylcellulose, Hydroxypropylcellulose, Hydroxypropylmethylcellulose oder Hydroxyethylcellulose, sowie kationische Cellulose-Derivate, z. B. die Handelsprodukte Celquat® und Polymer JR®, und bevorzugt Celquat® H 100, Celquat® L 200 und Polymer JR® 400 (Polyquaternium-10) sowie Polyquaternium-24. Weitere bevorzugte Beispiele sind Polysaccharide aus Fucose-Einheiten, z. B. das Handelsprodukt Fucogel®.
In den erfindungsgemäßen Zusammensetzungen sind die Mono-, Oligo- oder Polysaccharide oder deren Derivate in Mengen von 0,1 - 10 Gew.-%, bevorzugt 0,5 - 5 Gew.% und besonders bevorzugt 1,0 - 3 Gew.-%, jeweils bezogen auf die gesamte Zusammensetzung, enthalten.
In einer bevorzugten Ausführungsform der vorliegenden Erfindung können die kosmetischen Zusammensetzungen weiterhin mindestens eine α-Hydroxycarbonsäure oder α-Ketocarbonsäure oder deren Ester-, Lacton- oder Salzform enthalten. Geeignete α-Hydroxycarbonsäuren oder α-Ketocarbonsäuren sind ausgewählt aus Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren oder α-Ketocarbonsäuren oder ihre Derivate sind in Mengen von 0,1 - 10 Gew.-%, bevorzugt 0,5 - 5 Gew.-%, jeweils bezogen auf die gesamte Zusammensetzung, enthalten.
Vorteilhafterweise können die erfindungsgemäßen Zusammensetzungen zusätzlich zu dem verdickenden Polymer in Latexform mindestens ein weiteres synthetisches filmbildendes, emulsionsstabilisierendes, verdickendes oder adhäsives Polymer, ausgewählt aus Polymeren, die kationisch, anionisch, amphoter geladen oder nichtionisch sein können, enthalten. Besonders bevorzugte anionische Homo- und Copolymere sind hierbei unvernetzte und vernetzte Polyacrylsäuren. Dabei können Allylether von Pentaerythrit, von Sucrose und von Propylen bevorzugte Vernetzungsagentien sein. Solche Verbindungen sind zum Beispiel die Handelsprodukte Carbopol®. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind beispielsweise Pemulen® und die Carbopol®-Typen 954, 980, 1342 und ETD 2020 (ex B.F. Goodrich).
Vorteilhafterweise liegen die erfindungsgemäßen kosmetischen Zusammensetzungen in Form einer flüssigen oder festen Öl-in-Wasser-Emulsion, Wasser-in-Öl-Emulsion, Wasser-in-Öl-in-Wasser-Emulsion, Öl-in-Wasser-in-Öl-Emulsion, Mikroemulsion, PIT-Emulsion oder Pickering-Emulsion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor.
In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT'-Emulsionen bevorzugt sein, die einen mittleren Teilchendurchmesser von etwa 200 nm aufweisen.
Besonders bevorzugt sind Zusammensetzungen in Form von Mikroemulsionen oder PIT-Emulsionen, die transparent sind, das heißt die eine Trübung von weniger als 200 NTU, bevorzugt weniger als 100 NTU und besonders bevorzugt weniger als 50 NTU aufweisen.
In der Ausführungsform als Emulsion oder als tensidische Lösung, z. B. als Reinigungsmittel, enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov®68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls® PGPH) und Polyglyceryl-3-diisostearat (Handelsprodukt Lameform® TGI) sowie lineare und verzweigte C₈-C₃₀-Fettsäuren und deren Na-, K-, Ammonium-, Ca-, Mg- und Zn - Salze.
Die erfindungsgemäßen Mittel enthalten die Emulgatoren bevorzugt in Mengen von 0,1 bis 25 Gew.-%, insbesondere 0,5 - 15 Gew.-%, bezogen auf das gesamte Mittel.
Weiterhin können die erfindungsgemäßen Zusammensetzungen schäumende nichtionische, zwitterionische, anionische und kationische Tenside enthalten.
Beispiele für nichtionische Tenside sind
- alkoxylierte Fettsäurealkylester der Formel R¹CO-(OCH₂CHR²)ₓOR³, in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und x für Zahlen von 1 bis 20 steht,
- Anlagerungsprodukte von Ethylenoxid an Fettsäurealkanolamide und Fettamine,
- Fettsäure-N-alkylglucamide,
- C₈ - C₂₂-Alkylamin-N-oxide,
- Alkylpolygykoside entsprechend der allgemeinen Formel RO-(Z)ₓ wobei R für eine C₈- C₁₆-Alkylgruppe, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind solche Alkylpolyglykoside, bei denen R im wesentlichen aus C₈- und C₁₀-Alkylgruppen, im wesentlichen aus C₁₂- und C₁₄-Alkylgruppen, im wesentlichen aus C₈- bis C₁₆-Alkylgruppen oder im wesentlichen aus C₁₂- bis C₁₆-Alkylgruppen besteht.
   Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglykoside enthalten im Schnitt 1,1 bis 5, bevorzugt 1,1 bis 2,0 besonders bevorzugt 1,1 bis 1,8 Zuckereinheiten. Auch die alkoxylierten Homologen der genannten Alkylpolyglykoside können erfindungsgemäß eingesetzt werden. Diese Homologen können durchschnittlich bis zu 10 Ethylenoxidund/oder Propylenoxideinheiten pro Alkylglykosideinheit enthalten.
   Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.
   Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykoloder Polyglykolether-Gruppen, Ester-, Ether- und Amidgruppen sowie Hydroxylgruppen enthalten sein. Beispiele für geeignete schäumende Aniontenside sind, jeweils in Form der Natrium-, Kalium- und Ammonium- sowie der Mono-, Di- und Trialkanolammoniumsalze mit 2 bis 4 Kohlenstoffatomen in der Alkanolgruppe,
- Acylglutamate der Formel (I), 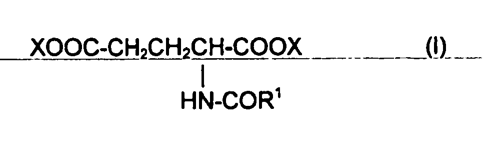 in der R¹CO für einen linearen oder verzweigten Acylrest mit 6 bis 22 Kohlenstoffatomen und 0, 1, 2 oder 3 Doppelbindungen und X für Wasserstoff, ein Alkaliund/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 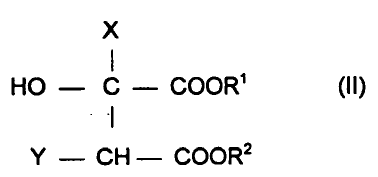 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, daß wenigstens eine der Gruppen R, R¹ oder R² ein Rest Z ist,
- Ester des Sulfobernsteinsäure-Salzes der allgemeinen Formel (III), 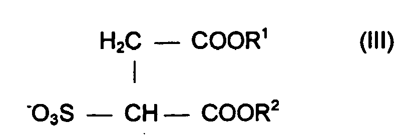 in der R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, daß wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Ethoxygruppen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcosinate mit einem linearen oder verzweigten Acylrest mit 6 bis 22 Kohlenstoffatomen und 0, 1, 2 oder 3 Doppelbindungen,
- Acyltaurate mit einem linearen oder verzweigten Acylrest mit 6 bis 22 Kohlenstoffatomen und 0, 1, 2 oder 3 Doppelbindungen,
- Acylisethionate mit einem linearen oder verzweigten Acylrest mit 6 bis 22 Kohlenstoffatomen und 0, 1, 2 oder 3 Doppelbindungen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)_{z}-SO₃X, in der R eine bevorzugt lineare Alkylgruppe mit 8 bis 30 C-Atomen, besonders bevorzugt mit 8 - 18 C-Atomen, z = 0 oder 1 bis 12, besonders bevorzugt 3, und X ein Natrium-, Kalium-, Magnesium-, Zink-, Ammoniumion oder ein Monoalkanol-, Dialkanol- oder Trialkanolammoniumion mit 2 bis 4 Kohlenstoffatomen in der Alkanolgruppe ist, wobei ein besonders bevorzugtes Beispiel Zinkcocoylethersulfat mit einem Ethoxylierungsgrad von z = 3 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Alkyl- und/oder Alkenyletherphosphate der Formel (IV) , 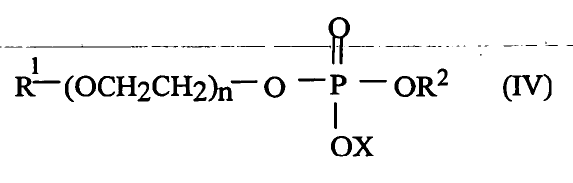 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für einen C₁ bis C₄ -Kohlenwasserstoffrest, steht,
- sulfatierte Fettsäurealkylenglykolester der Formel R⁷CO(AlkO)ₙSO₃M, in der R⁷COfür einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (V), 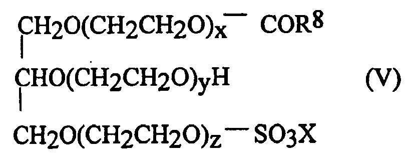
in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (V) eingesetzt, in der R⁸CO für einen linearen Acylrest mit 8 bis 18 Kohlenstoffatomen steht.

Weitere geeignete Zusatzstoffe sind Fettstoffe, insbesondere pflanzliche Öle, wie Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls, flüssige Paraffinöle, Isoparaffinöle und synthetische Kohlenwasserstoffe, Di-n-alkylether mit insgesamt 12 bis 36 C-Atomen, z. B. Di-n-octylether und n-Hexyl-n-octylether, Fettsäuren, besonders lineare und/oder verzweigte, gesättigte und/oder ungesättigte C₈₋₃₀-Fettsäuren, Fettalkohole, besonders gesättigte, ein- oder mehrfach ungesättigte, verzweigte oder unverzweigte Fettalkohole mit 6 - 30 Kohlenstoffatomen, Esteröle, das heißt Ester von C₆-₃₀-Fettsäuren mit C₂₋₃₀-Fettalkoholen, Hydroxycarbonsäurealkylester, wobei die Vollester der Glycolsäure, Milchsäure, Äpfelsäure, Weinsäure oder Citronensäure bevorzugt sind, Dicarbonsäureester wie Di-n-butyladipat sowie Diolester wie Ethylenglykoldioleat oder Propylenglykoldi(2-ethylhexanoat), symmetrische, unsymmetrische oder cyclische Ester der Kohlensäure mit Fettalkoholen, z. B. Glycerincarbonat oder Dicaprylylcarbonat (Cetiol® CC), Mono,- Di- und Trifettsäureester von gesättigten und/oder ungesättigten linearen und/oder verzweigten Fettsäuren mit Glycerin Wachse, insbesondere Insektenwachse, Pflanzenwachse, Fruchtwachse, Ozokerit, Mikrowachse, Ceresin, Paraffinwachse, Triglyceride gesättigter und gegebenenfalls hydroxylierter C₁₆₋₃₀-Fettsäuren, z. B. gehärtete Triglyceridfette, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxane, Polyalkylarylsiloxane, ethoxylierte Polydialkylsiloxane sowie Polydialkylsiloxane, die Aminund/oder Hydroxy-Gruppen enthalten.
Die Einsatzmenge der Fettstoffe beträgt 0,1 - 50 Gew.%, bevorzugt 0,1 - 20 Gew.% und besonders bevorzugt 0,1 - 15 Gew.%, jeweils bezogen auf das gesamte Mittel.

Die erfindungsgemäßen Mittel können weitere Wirk-, Hilfs- und Zusatzstoffe enthalten, beispielsweise Vitamine, Provitamine und Vitaminvorstufen aus den Gruppen A, B, C, E und F, Allantoin, Bisabolol und Antioxidantien.

In der erfindungsgemäßen Ausführungsform als Antitranspirant können Antitranspirant-Wirkstoffe enthalten sein. Als Antitranspirant-Wirkstoffe eignen sich erfindungsgemäß wasserlösliche adstringierende oder einweißkoagulierende metallische Salze, insbesondere anorganische und organische Salze des Aluminiums, Zirkoniums, Zinks und Titans sowie beliebige Mischungen dieser Salze. Erfindungsgemäß wird unter Wasserlöslichkeit eine Löslichkeit von wenigstens 4 g Aktivsubstanz pro 100 g Lösung bei 20 °C verstanden. Erfindungsgemäß verwendbar sind beispielsweise Alaun (KAI(SO₄)₂ · 12 H₂O), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat, Aluminium-Zirkonium-Chlorohydrat-Glycin-Komplexe und Komplexe von basischen Aluminiumchloriden mit Propylenglycol oder Polyethylenglycol. Bevorzugt enthalten die flüssigen Wirkstoffzubereitungen ein adstringierendes Aluminiumsalz, insbesondere Aluminiumchlorohydrat, und/ oder eine Aluminium-Zirkonium-Verbindung. Aluminiumchlorohydrate werden beispielsweise pulverförmig als Micro Dry® Ultrafine oder in aktivierter Form als Reach® 501 oder Reach® 103 von Reheis sowie in Form wäßriger Lösungen als Locron® L von Clariant oder als Chlorhydrol® von Reheis vertrieben. Unter der Bezeichnung Reach® 301 wird ein Aluminiumsesquichlorohydrat von Reheis angeboten. Auch die Verwendung von Aluminium-Zirkonium-Tri- oder Tetrachlorohydrex-Glycin-Komplexen, die beispielsweise von Reheis unter der Bezeichnung Rezal® 36G im Handel sind, ist erfindungsgemäß besonders vorteilhaft.
Der schweißhemmende Wirkstoff ist in den erfindungsgemäßen Zusammensetzungen in einer Menge von 0,01 - 40 Gew.-%, vorzugsweise 2 - 30 Gew.-% und insbesondere 5-25 Gew.-%, bezogen auf die Menge der Aktivsubstanz in der gesamten Zusammensetzung, enthalten.
In der erfindungsgemäßen Ausführungsform als Deodorant können Deodorant-Wirkstoffe enthalten sein. Erfindungsgemäß als Deodorant-Wirkstoffe geeignet sind Duftstoffe, antimikrobielle, antibakterielle oder keimhemmende Stoffe, enzymhemmende Stoffe, Antioxidantien und Geruchsadsorbentien.
Geeignete antimikrobielle, antibakterielle oder keimhemmende Stoffe sind insbesondere C₁-C₄-Alkanole, C₂-C₄-Alkandiole, Organohalogenverbindungen sowie -halogenide, quartäre Ammoniumverbindungen, eine Reihe von Pflanzenextrakten und Zinkverbindungen.

In einer weiteren bevorzugten Ausführungsform enthalten die erfindungsgemäßen Zusammensetzungen mindestens ein wasserlösliches Polyol, ausgewählt aus wasserlöslichen Diolen, Triolen und höherwertigen Alkoholen sowie Polyethylenglycolen. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Pentandiole, z. B. 1,2-Pentandiol, sowie Hexandiole, z. B. 1,6-Hexandiol. Weiterhin bevorzugt geeignet sind Glycerin und technische Oligoglyceringemische mit einem Eigenkondensationsgrad von 1,5 bis 10 wie etwa technische Diglyceringemische mit einem Diglyceringehalt von 40 bis 50 Gew.-% oder Triglycerin, weiterhin 1,2,6-Hexantriol sowie Polyethylenglycole (PEG) mit einem durchschnittlichen Molekulargewicht von 100 bis 1.000 Dalton, beispielsweise PEG-400, PEG-600 oder PEG-1000. Weitere geeignete höherwertige Alkohole sind die C₄-, C₅- und C₆-Monosaccharide und die entsprechenden Zuckeralkohole, z. B. Mannit oder Sorbit.
Die erfindungsgemäßen Zusammensetzungen enthalten das wasserlösliche Polyol in Mengen von 1 - 50 Gew.-%, bevorzugt 1 - 15 Gew.-% und besonders bevorzugt 1 - 5 Gew.-%, jeweils bezogen auf die gesamte Zusammensetzung.

Die folgenden Beispiele sollen die Erfindung verdeutlichen, ohne sie hierauf zu beschränken.

### Beispiele

| **Beispiel 1) O/W - Hautcreme zur Faltenbehandlung** | | |
|---|---|---|
| **Bestandteil** | INCI-Bezeichnung | Menge |
| Sojalecithin | | 0,50 |
| Isopropylstearat | | 2,00 |
| Myritol® 318 | Caprylic/Capric Triglyceride | 1,00 |
| Tocopherylacetat | | 0,50 |
| Cutina® MD-V | Glyceryl Stearate | 1,00 |
| Cetearylalkohol | | 1,50 |
| Dimethicone | | 5,00 |
| Propylparaben | | 0,20 |
| Weizenproteinhydrolysat | | 1,00 |
| Dow Corning 9040 | Cyclomethicone/Dimethicone Crosspolymer | 1,00 |
| Glycerin | | 5,00 |
| 1,6-Hexandiol | | 6,00 |
| Methylparaben | | 0,20 |
| Tego Carbomer 2%ig | Carbomer | 15,00 |
| Dimethylsilanol-Hyaluronat | | 0,20 |
| Algenextrakt | | 1,00 |
| 1,2-Propylenglycol | | 5,00 |
| Dimethylmethoxychromanol-6 | | 0,01 |
| Simulgel® NS | HYDROXYETHYL ACRYLATE/SODIUM ACRYLOYLDIMETHYLTAURA TE COPOLYMER, SQUALANE, POLYSORBATE 60 | 2,00 |
| NaOH 10%ige Lsg. | | 0,23 |
| Mica | | 3,00 |
| Wasser | | ad 100 |

| **Beispiel 2: Gel zur Behandlung von Fältchen** | | |
|---|---|---|
| Glycerin | | 10,00 |
| Na-Hyaluronsäure-Salz | | 0,2,00 |
| Dow Corning 9509 | Dimethicone/ Vinyldimethicone Crosspolymer and C₁₂ - C₁₄ Pareth-12 | 20,00 |
| Simulgel® NS | Hydroxyethyl Acrylate / Sodium Acryloyl Dimethyltaurate Copolymer, Squalane, Polysorbate 60 | 3,00 |
| Dow Corning 245 | Cyclopentasiloxane | 20,00 |
| Dow Corning - 9040 | Cyclomethicone/Dimethicone Crosspolymer | 5,00 |
| Phenonip® | Phenoxyethanol, Methylparaben, Ethylparaben, Propylparaben, Butylparaben | 0,50 |
| Mica | | 2,00 |
| Wasser | | ad 100 |

| **Beispiel 3) Shampoo** | | |
|---|---|---|
| **Bestandteil** | **INCI - Bezeichnung** | **Gew.-%** |
| TEXAPON® NSO | Sodium Laureth Sulfate | 40,0 |
| DEHYTON® G | Disodium Cocoampho-diacetate | 6,0 |
| Polymer JR® 400 | Polyquaternium-10 | 0,5 |
| Cetiol® HE | PEG-7 Glyceryl Cocoate | 3,0 |
| Citronensäure | Citric Acid | 0,5 |
| Natriumbenzoat | Sodium Benzoate | 0,5 |
| Natriumchlorid | Sodium Chloride | 0,5 |
| Parfüm | | 0,4 |
| Dow Corning-9040 | Cyclomethicone and Dimethicone Crosspolymer | 1,0 |
| Simulgel® NS | Hydroxyethyl Acrylate / Sodium Acryloyl Dimethyltaurate Copolymer, Squalane, Polysorbate 60 | 1,0 |
| Wasser | | ad 100 |

| **Beispiel 4) Haarspülung** | | |
|---|---|---|
| **Bestandteil** | **INCl-Bezeichnung** | **Gew.-%** |
| Eumulgin® B2 | Ceteareth-20 | 0,3 |
| Stenol® 1618 | Cetearyl Alcohol | 3,3 |
| Crodamol® IPM | Isopropyl Myristate | 0,5 |
| Paraffinöl DAB 9 | Paraffinum liquidum | 0,3 |
| Dehyquart®L 80 | Dicocoylethyl Hydroxyethyl-monium Methosulfate (and) Propylene Glycol | 0,4 |
| Cosmedia Guar® C 261 | Guar Hydroxypropyl-trimoniumchloride | 1,5 |
| Promois® Milk-CAQ | Cocodimonium Hydroxypropyl Hydrolyzed Casein | 3,0 |
| Baumwollsamenextrakt | Propylene Glycol (and) PEG-10 Olive Glycerides (and) Aqua (and) Gossypium | 1,0 |
| Citronensäure | Citric Acid | 0,4 |
| Phenonip® | Phenoxyethanol, Methylparaben, Ethylparaben, Propylparaben, Butylparaben | 0,8 |
| Dow Corning - 9040 | Cyclomethicone and Dimethicone Crosspolymer | 1,0 |
| Simulgel® NS | Hydroxyethyl Acrylate / Sodium Acryloyl Dimethyltaurate Copolymer, Squalane, Polysorbate 60 | 1,0 |
| Wasser | | ad 100 |

| **Beispiel 5) Haarkur** | | |
|---|---|---|
| **Bestandteil** | **INCI - Bezeichnung** | **Gew.-%** |
| Dehyquart® L80 | Dicocoylethyl Hydroxyethylmonium Methosulfate (and) Propylene Glycol | 2,0 |
| Rewoquat® W 575 PG | Quaternium-87, Propylene Glycol | 2,0 |
| Paraffinöl DAB 9 | Paraffinum liquidum | 2,0 |
| Stenol 1618 | Cetearyl Alcohol | 6,0 |
| Cosmedia Guar® C 261 | Guar Hydroxypropyl-trimoniumchloride | 0,5 |
| Simulgel® NS | Hydroxyethyl Acrylate / Sodium Acryloyl Dimethyltaurate Copolymer, Squalane, Polysorbate 60 | 1,0 |
| DC - 9040 | Cyclomethicone and Dimethicone Crosspolymer | 1,0 |
| Ajidew® NL 50 | Sodium PCA | 2,5 |
| Gluadin® W 20 | Aqua (and) Hydrolyzed Wheat Protein (and) Sodium Benzoate (and) Phenoxyethanol (and) Methylparaben (and) Propylparaben) | 3,0 |
| Citronensäure | Citric Acid | 0,15 |
| Phenonip® | Phenoxyethanol, Methylparaben, Ethylparaben, Propylparaben, Butylparaben | 0,8 |
| Wasser | | ad 100 |

| **Beispiel 6) Haarkur** | | |
|---|---|---|
| **Bestandteil** | **INCI - Bezeichnung** | **Gew.-%** |
| Dehyquart® L80 | Dicocoylethyl Hydroxyethylmonium Methosulfate (and) Propylene Glycol | 2,0 |
| Rewoquat® W 575 PG | Quaternium-87, Propylene Glycol | 2,0 |
| Paraffinöl DAB 9 | Paraffinum liquidum | 2,0 |
| Stenol 1618 | Cetearyl Alcohol | 6,0 |
| Cosmedia Guar® C 261 | Guar Hydroxypropyl-trimoniumchloride | 0,5 |
| Simulgel® NS | Hydroxyethyl Acrylate / Sodium Acryloyl Dimethyltaurate Copolymer, Squalane, Polysorbate 60 | 1,0 |
| Dow Corning - 9040 | Cyclomethicone and Dimethicone Crosspolymer | 1,0 |
| Ajidew® NL 50 | Sodium PCA | 2,5 |
| Gluadin® W 20 | Aqua (and) Hydrolized Wheat Protein (and) Sodium Benzoate (and) Phenoxyethanol (and) Methylparaben (and) Propylparaben) | 3,0 |
| Citronensäure | Citric Acid | 0,15 |
| Phenonip® | Phenoxyethanol, Methylparaben, Ethylparaben, Propylparaben, Butylparaben | 0,8 |
| Wasser | | ad 100 |

| **Beispiel 7) Haarkur** | | |
|---|---|---|
| **Bestandteil** | **INCI - Bezeichnung** | **Gew.-%** |
| Dehyquart® F75 | Distearoylethyl Hydroxyethylmonium Methosulfate (and) Cetearyl Alcohol | 0,3 |
| Simulgel® NS | Hydroxyethyl Acrylate / Sodium Acryloyl Dimethyltaurate Copolymer, Squalane, Polysorbate 60 | 1,0 |
| Dow Corning 9040 | Cyclomethicone and Dimethicone Crosspolymer | 1,0 |
| Dow Corning® 200 Fluid | Dimethicone | 1,5 |
| Gafquat® 755N | Polyquaternium-11 | 1,5 |
| Biodocarb® | lodopropynyl Butylcarbamate | 0,02 |
| Citronensäure | Citric Acid | 0,15 |
| Parfümöl | | 0,25 |
| Wasser | | ad 100 |

| **Beispiel 8) Antitranspirant-Gel** | | |
|---|---|---|
| Simulgel® NS | Hydroxyethyl Acrylate / Sodium Acryloyl Dimethyltaurate Copolymer, Squalane, Polysorbate 60 | 1,0 |
| Dow Corning - 9040 | Cyclomethicone and Dimethicone Crosspolymer | 1,0 |
| Abil® EM 97 | Bis-PEG/PPG-14/14 DIMETHICONE, CYCLOPENTASILOXANE | 1,0 |
| Dow Corning 245 Fluid | Cyclopentasiloxan, Cyclohexasiloxan | 20,0 |
| Propylenglykol -1,2 | | 21,9 |
| Ethanol | | 8,0 |
| Aluminium-hydroxydchlorid Lsg. 50% | | 40,00 |
| Wasser | | ad 100 |

## Patentansprüche

1. Kosmetische Zusammensetzung, enthaltend
a) mindestens ein Silicon-Elastomer, das erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist und
b) mindestens einen polymeren Verdicker in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten, wobei der Polyelektrolyt ausgewählt ist aus teilweise oder vollständig neutralisierten, vernetzten Copolymeren aus 2-Methyl-2[(1-oxo-2-propenyl)amino]-1-propansulfonsäure (AMPS) und Hydroxyethylacrylat, in einem geeigneten kosmetischen Träger.

2. Zusammensetzung gemäß einem der vorhergehenden Ansprüche, **dadurch gekennzeichnet, dass** das Polyelektrolytmonomer 2-Methyl-2[(1-oxo-2-propenyl)amino]-1-propansulfonsäure teilweise oder vollständig neutralisiert ist als Natrium-, Kalium-, Ammonium-, Ethanolamin- oder Aminosäure-Salz.

3. Zusammensetzung gemäß einem der vorhergehenden Ansprüche, **dadurch gekennzeichnet, dass** die Ölphase des inversen Polymerlatex mindestens ein Öl, ausgewählt aus weißen Mineralölen, Squalan und hydriertem Polyisobuten, enthält.

4. Zusammensetzung gemäß einem der vorhergehenden Ansprüche, **dadurch gekennzeichnet, dass** der in dem inversen Polymerlatex enthaltene Öl-in-Wasser-Emulgator ausgewählt ist aus den Ethylenoxid-Addukten von Sorbitanoleat, Ricinusöl, das gewünschtenfalls gehärtet ist, Sorbitanlaurat und Laurylalkohol.

5. Zusammensetzung gemäß einem der vorhergehenden Ansprüche, **dadurch gekennzeichnet, dass** das Silicon-Elastomer ausgewählt ist aus nicht-emulgierenden und emulgierenden Silicon-Elastomeren.

6. Zusammensetzung gemäß einem der vorhergehenden Ansprüche, **dadurch gekennzeichnet, dass** das Organopolysiloxan mit mindestens 2 C₂ - C₁₀-Alkenyl-Gruppen mit terminaler Doppelbindung im Molekül ausgewählt ist aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

7. Zusammensetzung gemäß einem der vorhergehenden Ansprüche, **dadurch gekennzeichnet, dass** das vernetzende Organopolysiloxan mit mindestens zwei Silicongebundenen Wasserstoffatomen ausgewählt ist aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-Siloxan-Copolymeren.

8. Verwendung einer kosmetischen Zusammensetzung gemäß einem der Ansprüche 1 - 7 zur nicht-therapeutischen, kosmetischen Behandlung und/oder Minimierung von Hautfalten und -fältchen.

9. Verwendung einer kosmetischen Zusammensetzung gemäß einem der Ansprüche 1 bis 7 zur pflegenden, verformenden, färbenden oder bleichenden Behandlung der Haare.

10. Verwendung einer kosmetischen Zusammensetzung gemäß einem der Ansprüche 1 bis 7 zur desodorierenden und/oder schweißhemmenden Behandlung der Haut, insbesondere der Achselhöhlen.

## Claims

1. A cosmetic composition comprising
a) at least one silicone elastomer, which may be obtained by crosslinking of an organopolysiloxane, which contains at least two C₂-C₁₀ alkenyl groups with a terminal double bond in each molecule, with an organopolysiloxane, which has at least two silicone-bound hydrogen atoms in each molecule and
b) at least one polymeric thickener in the form of an inverse, self-inversible polymer latex, containing an oil phase, a water phase, at least one oil-in-water emulsifier and at least one branched or cross-linked polyelectrolyte, wherein the polyelectrolyte is selected from partly or fully neutralized cross-linked copolymers of 2-methyl-2[(1-oxo-2-propenyl)amino]-1-propane-sulfonic acid (AMPS) and hydroxyethyl acrylate,
in a suitable cosmetic carrier.

2. The composition according to any of the preceding claims, **characterized in that** the polyelectrolyte monomer 2-methyl-2[(1-oxo-2-propenyl)amino]-1-propane-sulfonic acid is partly or fully neutralized in the form of a sodium, potassium, ammonium, ethanolamine or amino acid salt.

3. The composition according to any of the preceding claims, **characterized in that** the oil phase of the inverse polymer latex contains at least one oil selected from white mineral oils, squalane and hydrogenated polyisobutene.

4. The composition according to any of the preceding claims, **characterized in that** the oil-in-water emulsifier contained in the inverse polymer latex is selected from ethylene oxide adducts of sorbitan oleate, castor oil, which is hardened if desired, sorbitan laurate and lauryl alcohol.

5. The composition according to any of the preceding claims, **characterized in that** the silicone elastomer is selected from non-emulsifying and emulsifying silicone elastomers.

6. The composition according to any of the preceding claims, **characterized in that** the organopolysiloxane with at least 2 C₂-C₁₀ alkenyl groups with terminal double bond in the molecule is selected from methylvinylsiloxanes, methylvinylsiloxane-dimethylsiloxane copolymers, dimethylpolysiloxanes with dimethylvinylsiloxy end groups, dimethylsiloxane-methylphenylsiloxane copolymers with dimethylvinylsiloxy end groups, dimethylsiloxane-diphenylsiloxane-methylvinylsiloxane copolymers with dimethylvinylsiloxy end groups, dimethylsiloxane-methylvinylsiloxane copoymers with trimethylsiloxy end groups, dimethylsiloxane-methylphenylsiloxane-methylvinylsiloxane copolymers with trimethylsiloxy end groups, methyl-(3,3,3-trifluoropropyl)-polysiloxanes with dimethylvinylsiloxy end groups and dimethylsiloxane-methyl-(3,3,3-trifluoropropyl)-siloxane copolymers with dimethylvinylsiloxy end groups;

7. The composition according to any of the preceding claims, **characterized in that** the crosslinking organopolysiloxane with at least two silicone-bound hydrogen atoms is selected from methylhydrogenpolysiloxane with trimethylsiloxy end groups, dimethylsiloxane-methylhydrogensiloxane copolymers with trimethylsiloxy end groups and cyclic dimethylsiloxane-methylhydrogen-siloxane copolymers.

8. The use of a cosmetic composition according to any of claims 1 to 7, for non-therapeutic cosmetic treatment and/or minimization of skin folds and wrinkles.

9. The use of a cosmetic composition according to any of claims 1 to 7, for hair-care, shaping, dying or bleaching treatment of hair.

10. The use of a cosmetic composition according to any of claims 1 to 7, for deodorizing and/or anti-perspirant treatment of skin, in particular of armpits.

## Revendications

1. Composition cosmétique, contenant
a) au moins un élastomère de silicone, qui peut être obtenu par réticulation d'un organopolysiloxane, qui contient au moins deux groupes C₂-C₁₀-alcényle avec une double liaison terminale dans chaque molécule, avec un organopolysiloxane qui présente au moins deux atomes d'hydrogène liés au silicone dans chaque molécule et
b) au moins un épaississant polymère sous forme d'un latex polymère inverse, auto-inversible, contenant une phase huileuse, une phase aqueuse, au moins un émulsifiant huile-dans-eau et au moins un polyélectrolyte ramifié ou réticulé, le polyélectrolyte étant choisi parmi les copolymères partiellement ou totalement neutralisés, réticulés d'acide 2-méthyl-2[(1-oxo-2-propényl)amino]-1-propanesulfonique (AMPS) et d'acrylate d'hydroxyéthyle,
dans un support cosmétique approprié.

2. Composition selon l'une quelconque des revendications précédentes, **caractérisée en ce que** le monomère du polyélectrolyte, l'acide 2-méthyl-2[(1-oxo-2-propényl)amino]-1-propanesulfonique, est partiellement ou totalement neutralisé sous forme de sel de sodium, de potassium, d'ammonium, d'éthanolamine ou d'aminoacide.

3. Composition selon l'une quelconque des revendications précédentes, **caractérisée en ce que** la phase huileuse du latex polymère inverse contient au moins une huile choisie parmi les huiles minérales blanches, le squalane et le polyisobutène hydrogéné.

4. Composition selon l'une quelconque des revendications précédentes, **caractérisée en ce que** l'émulsifiant huile-dans-eau contenu dans le latex polymère inverse est choisi parmi les produits d'addition d'oxyde d'éthylène sur de l'oléate de sorbitane, de l'huile de ricin qui est si souhaité durcie, du laurate de sorbitane et de l'alcool laurylique.

5. Composition selon l'une quelconque des revendications précédentes, **caractérisée en ce que** l'élastomère de silicone_est choisi parmi les élastomères de silicone_non émulsifiants et émulsifiants.

6. Composition selon l'une quelconque des revendications précédentes, **caractérisée en ce que** l'organopolysiloxane présentant au moins deux groupes C₂-C₁₀-alcényle avec une double liaison terminale dans la molécule est choisi parmi les méthylvinylsiloxanes, les copolymères de méthylvinylsiloxane-diméthylsiloxane, les diméthylpolysiloxanes avec des groupes terminaux diméthylvinylsiloxy, les copolymères de diméthylsiloxane-méthylphénylsiloxane avec des groupes terminaux diméthylvinylsiloxy, les copolymères de diméthylsiloxane-diphénylsiloxane-méthylvinylsiloxane avec des groupes terminaux diméthylvinylsiloxy, les copolymères de diméthylsiloxane-méthylvinylsiloxane avec des groupes terminaux triméthylsiloxy, les copolymères de diméthylsiloxane-méthylphénylsiloxane-méthylvinylsiloxane avec des groupes terminaux triméthylsiloxy, les méthyl-(3,3,3-trifluoropropyl)-polysiloxanes avec des groupes terminaux diméthylvinylsiloxy et les copolymères de diméthylsiloxane-méthyl-(3,3,3-trifluoropropyl)-siloxane avec des groupes terminaux diméthylvinylsiloxy.

7. Composition selon l'une quelconque des revendications précédentes, **caractérisée en ce que** l'organopolysiloxane réticulant présentant au moins deux atomes d'hydrogène liés au silicone est choisi parmi les méthylhydrogénopolysiloxanes avec des groupes terminaux triméthylsiloxy, les copolymères de diméthylsiloxane-méthylhydrogénosiloxane avec des groupes terminaux triméthylsiloxy et les copolymères cycliques de diméthylsiloxane-méthylhydrogénosiloxane.

8. Utilisation d'une composition cosmétique selon l'une quelconque des revendications 1 à 7 pour le traitement non thérapeutique, cosmétique et/ou la minimisation de rides et de ridules de la peau.

9. Utilisation d'une composition cosmétique selon l'une quelconque des revendications 1 à 7 pour le traitement de soin, de déformation, de coloration ou de décoloration des cheveux.

10. Utilisation d'une composition cosmétique selon l'une quelconque des revendications 1 à 7 pour le traitement désodorisant et/ou inhibant la transpiration de la peau, en particulier des creux des aisselles.
